# EUROPEAN PATENT SPECIFICATION

(11) **EP 1 551 988 B1**
(45) Date of publication and mention of the grant of the patent: **24.11.2010**
(21) Application number: 03765839.0
(22) Date of filing: 21.07.2003
(51) Int. Cl.: C12P 21/00, C12P 21/04, C12N 15/00, C12N 15/63, C12N 15/87, C12Q 1/68, C12Q 1/70, G01N 33/53, A61K 48/00

(54) **METHODS FOR GENERATING ENHANCED ANTIBODY-PRODUCING CELL LINES WITH IMPROVED GROWTH CHARACTERISTICS**
VERFAHREN ZUR ERZEUGUNG VERBESSERTER ANTIKöRPER PRODUZIERENDER ZELLINIEN MIT VERBESSERTEN WACHSTUMSEIGENSCHAFTEN
PROCEDES PERMETTANT DE GENERER DES LIGNEES CELLULAIRES PRODUCTRICES D'ANTICORPS RENFORCEES PRESENTANT DES CARACTERISTIQUES DE CROISSANCE AMELIOREES

(30) Priority: 19.07.2002 US 397027 P
(43) Date of publication of application: 13.07.2005
(73) Proprietor: Morphotek, Inc., Exton, PA 19341 (US)
(72) Inventor: GRASSO, Luigi, Pennsylvania, PA 19010 (US); KLINE, J. Bradford, Norristown, PA 19403 (US); NICOLAIDES, Nicholas, C., Pennsylvania, PA 19061 (US); SASS, Philip, M., Audubon, PA 19403 (US)
(74) Representative: W.P. Thompson & Co.
(86) International application number: PCT/US2003/022743
(87) International publication number: WO 2004/009782

(56) References cited:
- WO-A-01/42485
- WO-A-02/37967
- WO-A-87/05626
- WO-A-98/45411
- WO-A-02/054856
- US-A1- 2002 055 106
- NICOLAIDES NICHOLAS C ET AL: "A naturally occurring hPMS2 mutation can confer a dominant negative mutator phenotype" MOLECULAR AND CELLULAR BIOLOGY, AMERICAN SOCIETY FOR MICROBIOLOGY, WASHINGTON, US, vol. 18, no. 3, March 1998 (1998-03), pages 1635-1641, XP002165242 ISSN: 0270-7306
- KIM N ET AL: "The role of DNA repair in somatic hypermutation of immunoglobulin genes" JOURNAL OF EXPERIMENTAL MEDICINE, TOKYO, JP, vol. 187, no. 11, 1 June 1998 (1998-06-01), pages 1729-1733, XP002978604 ISSN: 0022-1007
- KIM N S ET AL: "Overexpression of bcl-2 inhibits sodium butyrate-induced apoptosis in Chinese hamster ovary cells resulting in enhanced humanized antibody production" BIOTECHNOLOGY AND BIOENGINEERING - COMBINATORIAL CHEMISTRY, WILEY, NEW YORK, NY, US, vol. 71, no. 3, 2000, pages 184-193, XP002208594
- NICOLAIDES N C ET AL: "MORPHOGENICS AS A TOOL FOR TARGET DISCOVERY AND DRUG DEVELOPMENT" ANNALS OF THE NEW YORK ACADEMY OF SCIENCES, NEW YORK ACADEMY OF SCIENCES, NEW YORK, NY, US, vol. 1059, 2005, pages 86-96, XP009071240 ISSN: 0077-8923
- WINTER D.B. ET AL: 'Altered spectra of hypermutation in DNA repair-deficient mice' PHILOSOPHICAL TRANSACTIONS OF THE ROYAL SOCIETY OF LONDON B vol. 356, 2001, pages 5 - 11, XP002978603
- KIM N. ET AL: 'The role of DNA repair in somatic hypermutation of immunoglobulin genes' J. EXP. MED. vol. 187, no. 11, 1998, pages 1729 - 1733, XP002978604
- SIFERS R.N. ET AL: 'A frameshift mutation results in a truncated alpha-1 antitrypsin that is retained within the rough endoplasmic reticulum' J. BIOL. CHEM. vol. 263, no. 15, 25 May 1988, pages 7330 - 7335, XP002978605
- SCOBIE G. ET AL: 'Non-specific effects of anti-sense oligonucleotides on alpha-1-antitrypsin expression in Cos cells transfected with alpha-1-antitrypsin cDNA constructs' BIOCHEMICAL SOCIETY TRANSACTIONS vol. 20, no. 3, 1992, page 319S, XP002978916
- OZAKI L. ET AL: 'Inhibition of alpha-1-antitrypsin (alpha-1-AT) gene expression by a hammerhead ribozyme in hepatoma cells' GASTROENTEROLOGY vol. 110, no. 4, 1996, page A1288, XP002978663

## Description

### TECHNICAL FIELD OF THE INVENTION

The invention is related to the area of antibody and recombinant protein production. In particular, it is related to the field of mutagenesis, gene discovery and recombinant gene expression.

### BACKGROUND OF THE INVENTION

The use of antibodies to block the activity of foreign and/or endogenous polypeptides provides an effective and selective strategy for treating the underlying cause of disease. In particular is the use of monoclonal antibodies (MAb) as effective therapeutics such as the FDA approved ReoPro (Glaser, V. (1996) "Can ReoPro repolish tarnished monoclonal therapeutics?" Nat. Biotechnol. 14:1216-1217), an anti-platelet MAb from Centocor; Herceptin (Weiner, L.M. (1999) "Monoclonal antibody therapy of cancer" Semin. Oncol. 26:43-51), an anti-Her2/neu MAb from Genentech; and Synagis (Saez-Llorens, X.E., et al. (1998) "Safety and pharmacokinetics of an intramuscular humanized monoclonal antibody to respiratory syncytial virus in premature infants and infants with bronchopulmonary dysplasia" Pediat. Infect. Dis. J. 17:787-791), an anti-respiratory syncytial virus MAb produced by Medimmune.

Standard methods for generating MAbs against candidate protein targets are known by those skilled in the art. Briefly, primates as well as rodents, such as mice or rats, are injected with a purified antigen in the presence of adjuvant to generate an immune response (Shield, C.F., et al. (1996) "A cost-effective analysis of OKT3 induction therapy in cadaveric kidney transplantation" Am. J. Kidney Dis. 27:855-864). Animals with positive immune sera are sacrificed and splenocytes are isolated. Isolated splenocytes are fused to myelomas to produce immortalized cell lines that are then screened for antibody production. Positive lines are isolated and characterized for antibody production. The direct use of rodent-derived MAbs as human therapeutic agents were confounded by the fact that human anti-rodent antibody (HARA) responses occurred in a significant number of patients treated with the rodent-derived antibody (Khazaeli, M.B., et al., (1994) "Human immune response to monoclonal antibodies" J. Immunother. 15:42-52). In order to circumvent the problem of HARA, the grafting of the complementarity determining regions (CDRs), which are the critical motifs found within the heavy and light chain variable regions of the immunoglobulin (Ig) subunits making up the antigen binding domain, onto a human antibody backbone found these chimeric molecules to retain their binding activity to antigen while lacking the HARA response (Emery, S.C., and Harris, W.J. "Strategies for humanizing antibodies" In: ANTIBODY ENGINEERING C.A.K. Borrebaeck (Ed.) Oxford University Press, N.Y. 1995. pp. 159-183. A common problem that exists during the "humanization" of rodent-derived MAbs (referred to hereon as HAb) is the loss of binding affinity due to conformational changes in the three-dimensional structure of the CDR domain upon grafting onto the human Ig backbone (U.S. Patent No. 5,530,101 to Queen et al.). To overcome this problem, additional HAb vectors are usually needed to be engineered whereby inserting or deleting additional amino acid residues within the framework region and/or within the CDR coding region itself in order to recreate high affinity HAbs (U.S. Patent No. 5,530,101 to Queen et al.). This process is a very time consuming procedure that involves the use of expensive computer modeling programs to predict changes that may lead to a high affinity HAb. In some instances the affinity of the HAb is never restored to that of the MAb, rendering them of little therapeutic use.

A problem that exists in antibody engineering is the generation of stable high yielding producer cell lines that is required for manufacturing of the molecule for clinical materials. Several strategies have been adopted in standard practice by those skilled in the art to circumvent this problem. One method is the use of Chinese Hamster Ovary (CHO) cells transfected with exogenous Ig fusion genes containing the grafted human light and heavy chains to produce whole antibodies or single chain antibodies, which are a chimeric molecule containing both light and heavy chains that form an antigen-binding polypeptide (Reff, M.E. (1993) "High-level production of recombinant immunoglobulins in mammalian cells" Curr. Opin. Biotechnol. 4:573-576).

Another method employs the use of human lymphocytes derived from transgenic mice containing a human grafted immune system or transgenic mice containing a human Ig gene repertoire. Yet another method employs the use of monkeys to produce primate MAbs, which have been reported to lack a human anti-monkey response (Neuberger, M., and Gruggermann, M. (1997) "Monoclonal antibodies: Mice perform a human repertoire" Nature 386:25-26). In all cases, the generation of a cell line that is capable of generating sufficient amounts of high affinity antibody poses a major limitation for producing sufficient materials for clinical studies. Because of these limitations, the utility of other recombinant systems such as plants are currently being explored as systems that will lead to the stable, high-level production of humanized antibodies (Fiedler, U., and Conrad, U. (1995) "High-level production and long-term storage of engineered antibodies in transgenic tobacco seeds" Bio/Teclmology 13:1090-1093).

WO 02/37967 discloses methods for generating genetically altered antibody-producing cell lines by introducing into antibody producing cells a dominant negative allele of a mismatch repair gene.

A method for generating genetically altered host cells either surrogate mammalian cells such as but not limited to SP20, NS0, CHO, *etc.* that are capable of secreting increased amounts of antibody will provide a valuable method for creating cell hosts for product development as well as allow for the generation of reagents useful for the discovery of downstream genes whose altered structure or expression levels when altered result in enhanced MAb production. The invention described herein is directed to the creation of genetically altered cell hosts with increased antibody production via the blockade of MMR that can in turn be used to screen and identify altered gene loci for directed alteration and generation of high titer production strains.

The invention facilitates the generation of high titer production of cell lines with elevated levels of antibody production for manufacturing as well as use for target discovery of genes involved in over-production of antibodies either a the gene expression level, processing level or secretion level. Other advantages of the present invention are described in the examples and figures described herein.

### SUMMARY OF THE INVENTION

The invention provides an in vitro or ex vivo method for producing a high titer antibody producing cell comprising the step of suppressing the expression of at least one gene involved in antibody production, wherein said gene comprises alpha 1-anti-trypsin.

The antibody producing cells suitable for use in the invention include, but are not limited to rodent, primate, human hybridomas or lymphoblastoids; mammalian cells transfected and expressing exogenous Ig light and/or heavy chains or chimeric single chain molecules; plant cells, yeast or bacteria transfected and expressing exogenous Ig light or heavy chains, or chimeric single chain molecules.

The cells that are capable of producing antibodies for use in the invention include cells that naturally produce antibodies, and cells that are engineered to produce antibodies through the introduction of immunoglobulin heavy and/or light chain encoding sequences.

In the methods of the invention said step of suppressing the expression of said alpha-1-antitrypsin gene comprises any one of:
a) introducing an antisense oligonucleotide directed against said alpha-1-antitrypsin gene;
b) introducing an expression vector comprising an antisense transcript directed against said alpha-1-antitrypsin gene;
c) introducing a knock out targeting vector to disrupt the endogenous function of said alpha-1-antitrypsin gene, or
d) introducing a polynucleotide comprising a ribozyme directed against said alpha-1-antitrypsin gene.

In the methods of the invention the suppression of the expression of said alpha-1-antitrypsin gene may alternatively be achieved by introducing intracellular blocking antibodies against the product of said alpha-1-antitrypsin gene, or by incubating cells with neutralizing antibody or derivates thereof directed against the product of said gene in the growth medium.

The methods of the invention may further additionally comprise the step of suppressing the expression of the endothelial monocyte-activating polypeptide I gene.

In the methods of the invention said step of suppressing the expression of the endothelial monocyte-activating polypeptide I gene may comprise any one of:
a) introducing an antisense oligonucleotide directed against said endothelial monocyte-activating polypeptide 1 gene;
b) introducing an expression vector comprising an antisense transcript directed against said endothelial monocyte-activating polypeptide I gene;
c) introducing a knock out targeting vector to disrupt the endogenous function of said endothelial monocyte-activating polypeptide I, or
d) introducing a polynucleotide comprising a ribozyme directed against said endothelial monocyte-activating polypeptide I gene.

In the methods of the invention the suppression of the expression of said endothelial monocyte-activating polypeptide I gene may alternatively be achieved by introducing intracellular blocking antibodies against the product of said endothelial monocyte-activating polypeptide I gene, or by incubating cells with neutralizing antibody or derivates thereof directed against the product of said gene in the growth medium.

Cells for use in the methods of the invention may comprise a hybridoma, an epithelial cell, ovarian, a kidney cell, a myeloid cell or a lymphoid cell.

Using comparative gene expression analysis between parental and hypermutable MAb over-producer cell lines, two genes (SEQ ID NO:1 and SEQ ID NO:2) were identified in an over-producer subclone to have significantly lower expression than the parental precursor line. Antisense expression constructs were prepared and antisense vectors were introduced into parental and assayed for enhanced MAb production. Blockade of expression of both genes resulted in significantly higher MAb production.

The invention also provides a host cell which produces antibody molecules or fragments thereof comprising a defect in the alpha-1-antitrypsin gene such that expression of alpha-1-antitrypsin is inhibited.

In the host cells of the invention said defect may comprise a deletion of the alpha-1-antitrypsin or a frameshift mutation in the alpha-1-antitrypsin gene.

Alternatively the host cells of the invention may comprise:
a) an expression vector comprising an antisense transcript of the alpha-1-antitrypsin gene whereby expression of said antisense transcript suppresses the expression of the alpha-1-antitrypsin gene;
b) a ribozyme that disrupts expression of the alpha-1-antitrypsin gene, or
c) an intracellular neutralizing antibody against the alpha-1-antitrypsin protein whereby said antibody suppresses the activity of alpha-1-antitrypsin.

The host cells of the invention may further comprise an expression vector comprising a polynucleotide sequence encoding at least a portion of an antibody molecule. The polynucleotide may encode at least an immunoglobulin light chain or fragment thereof, or at least an immunoglobulin heavy chain or fragment thereof.

The host cells of the invention may further comprise a defect in the endothelial monocyte-activating polypeptide I gene such that expression of monocyte-activating polypeptide I is inhibited.

In the host cells of the invention comprising a defect in the endothelial monocyte-activating polypeptide I gene the defect may comprise a deletion of the endothelial monocyte-activating polypeptide I or a frameshift mutation in the endothelial monocyte-activating polypeptide I gene.

Alternatively, in the host cells of the invention comprising a defect in the endothelial monocyle-activating polypeptide I gene the host may comprise:
a) an expression vector comprising an antisense transcript of the endothelial monocyte-activating polypeptide I gene whereby expression of said antisense transcript suppresses the expression of the endothelial monocyte-activating polypeptide I gene;
b) a ribozyme that disrupts expression of the endothelial monocyte-activating polypeptide I gene; or
c) an intracellular neutralizing antibody against the endothelial monocyte-activating polypeptide I protein whereby said antibody suppresses the activity of endothelial monocyte-activating polypeptide I.

The invention is based on the discovery of two genes (SEQ ID NO:1 and SEQ ID NO:2) whose suppressed expression results in enhanced antibody production. Expression analysis of said genes are found to be significantly lower in over-producer sublines as compared to parental lines. Said genes expression are suppressed in parental lines and lines are screened for antibody production. Lines with inhibited expression of genes have enhanced antibody production. The cell lines may be bacterial, yeast, plant or mammalian cells including, but not limited to rabbit cells, rodent cells (*e.g*., mouse, rat, hamster), and primate cells (including human cells).

### BRIEF DESCRIPTION OF THE DRAWINGS

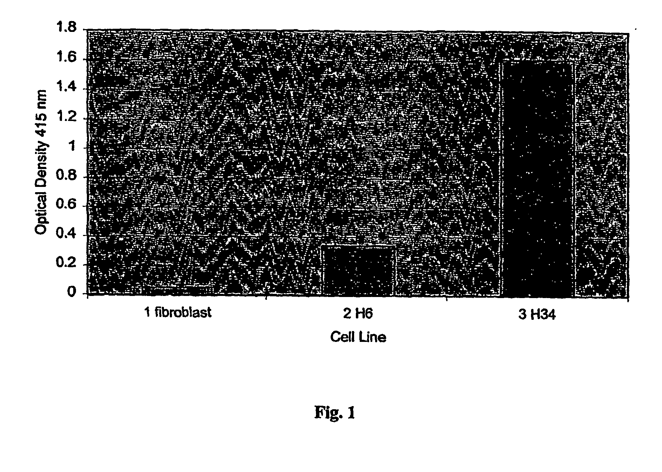

**Figure 1** shows the generation of MMR-defective clones with enhanced steady state antibody levels. An ELISA was carried out measuring antibody yields from 5 day old cultures of 10,000 cells from MMR defective H34 hybridoma clones with enhanced antibody titer yields (>500ngs/ml) within the conditioned medium as compared to the parental H6 cell line. Lane 1: fibroblast cells (negative control); Lane 2: H6 cell; Lane 3: H34 high titer line.

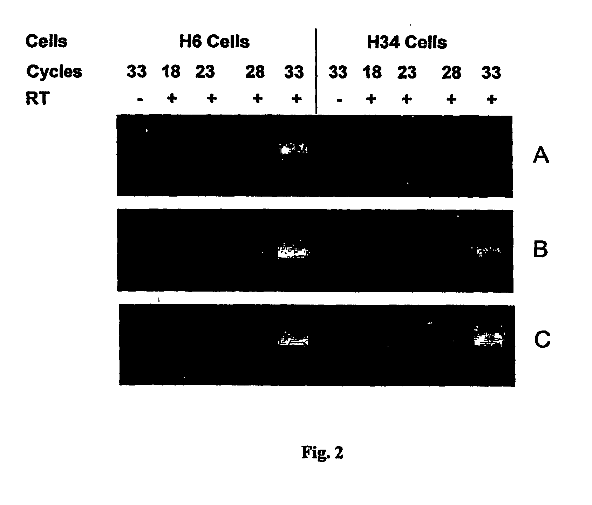

**Figure 2** shows expression Analysis of Immunoglobulin Enhancer Genes. RT-PCR validating the reduced expression of AAT (panel A) and EMAPI (panel B). RNAs were reverse transcribed from H6 parental and H34 enhanced producer clones and PCR amplified for AAT (panel A), EMAPI (panel B), and dihydrofolate reductase (DHFR) (panel C) which served as control. Samples were amplified for varying cycles to measure steady-state expression. The minus lane was RNTA process without reverse transcriptase which served as a negative control.

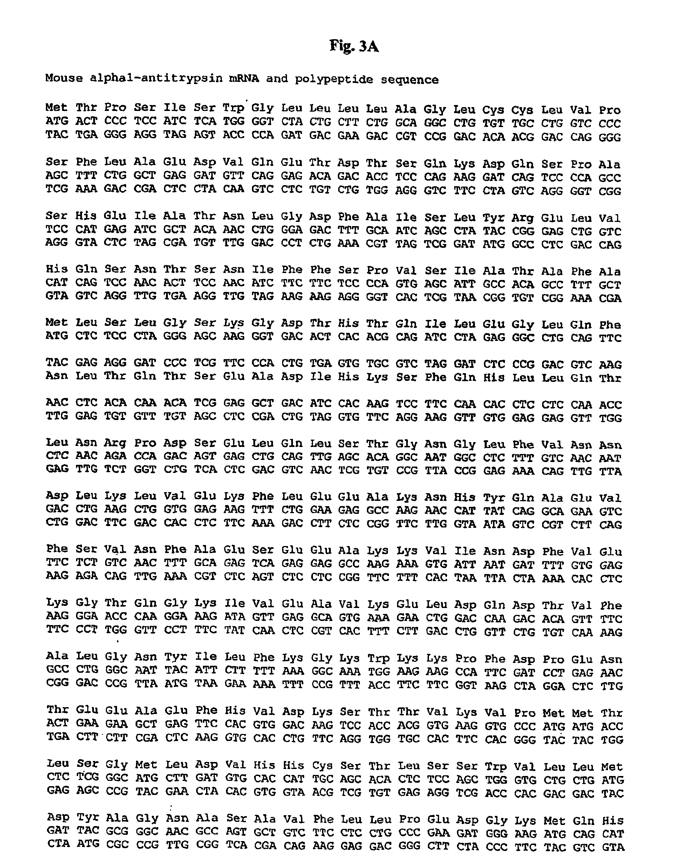

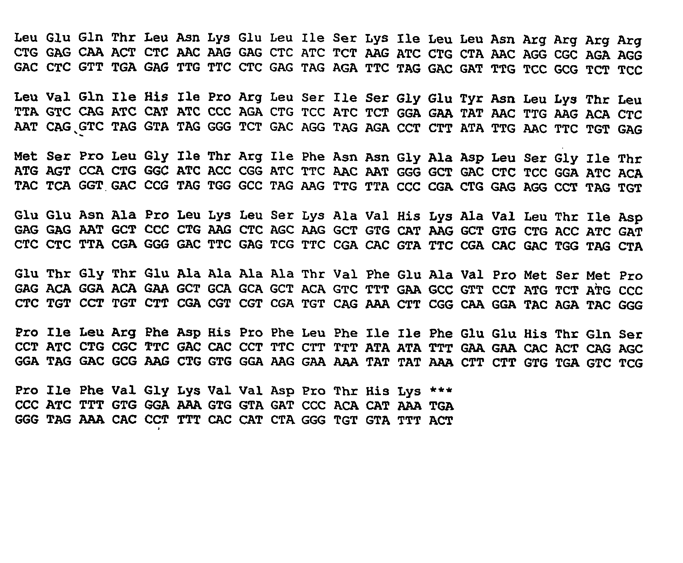

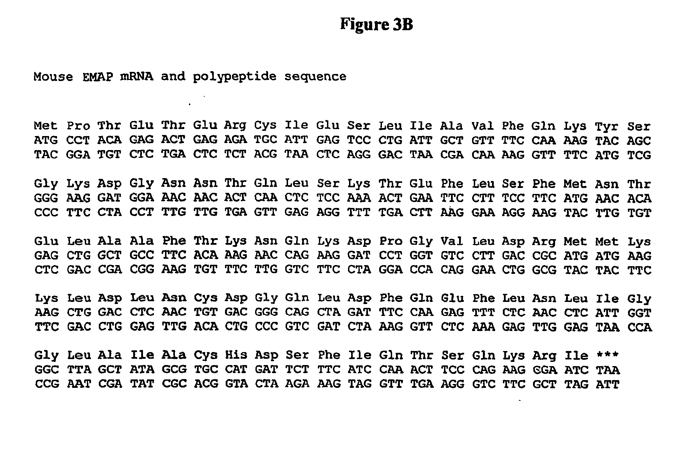

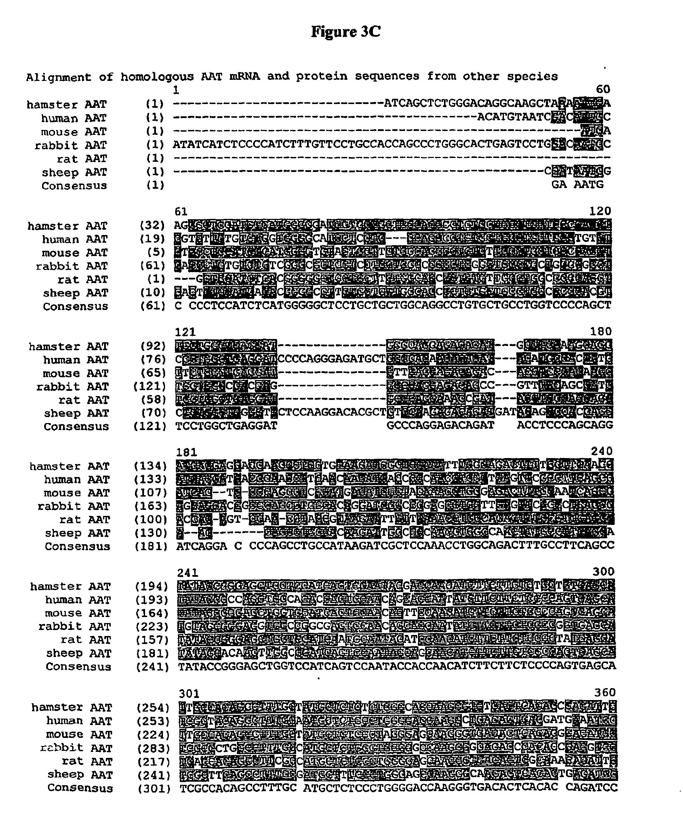

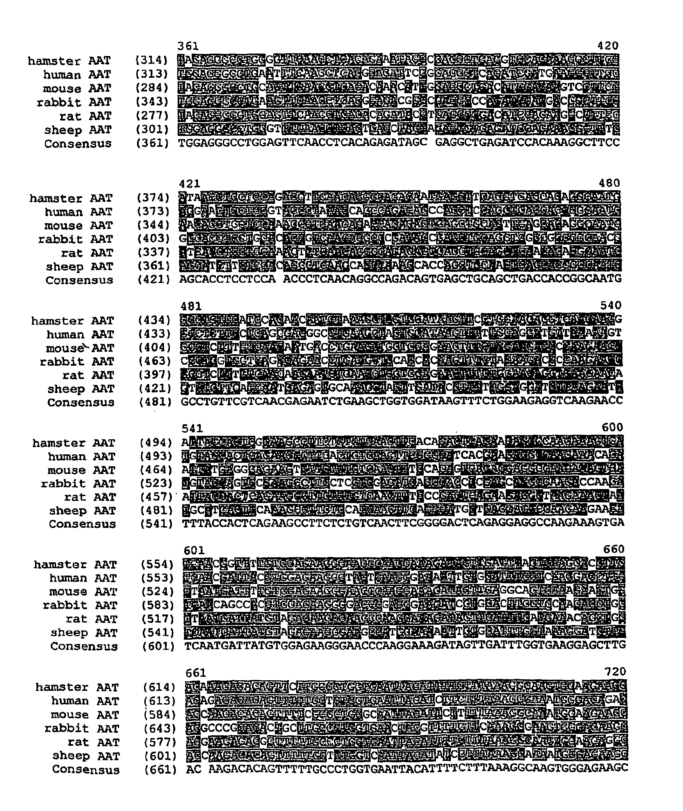

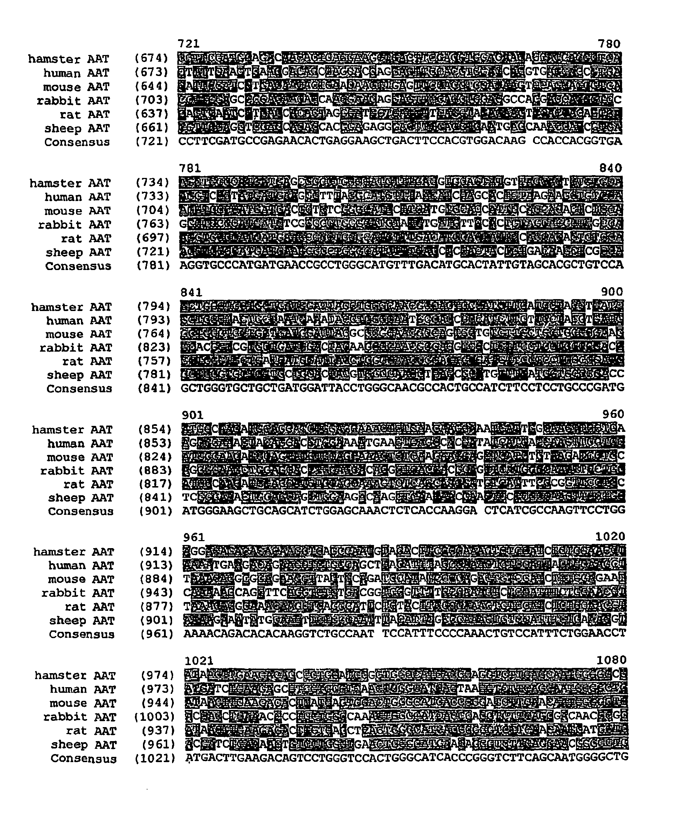

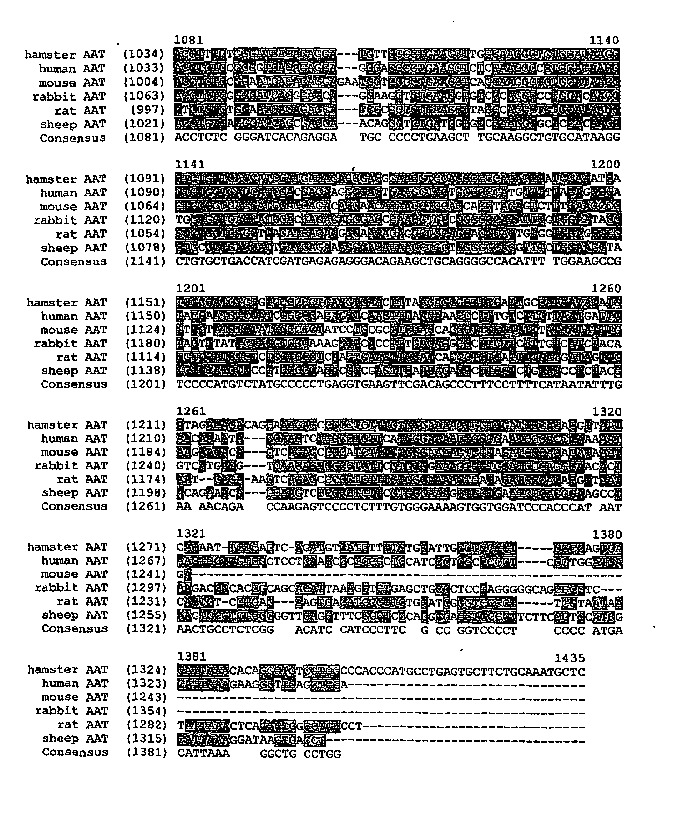

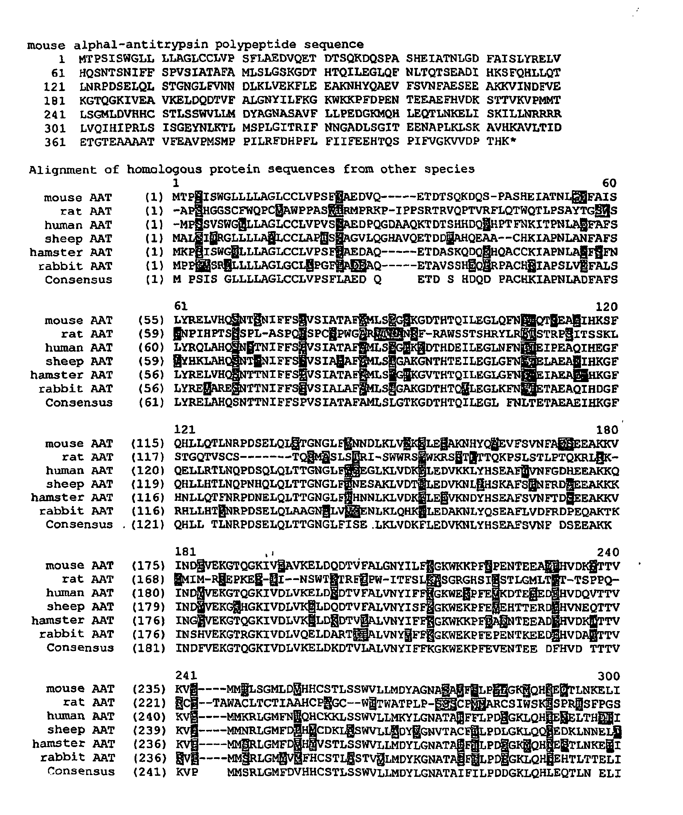

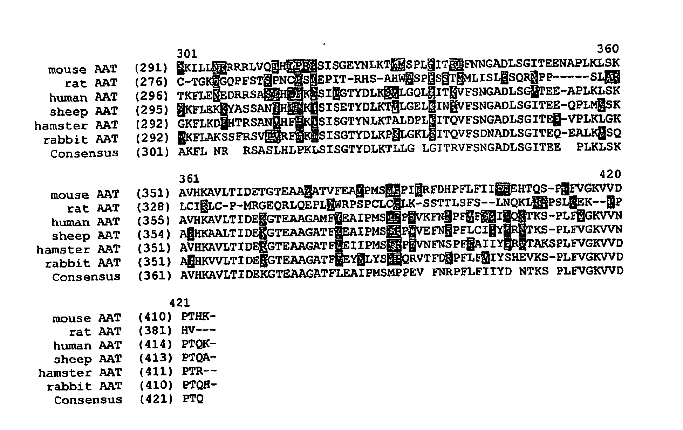

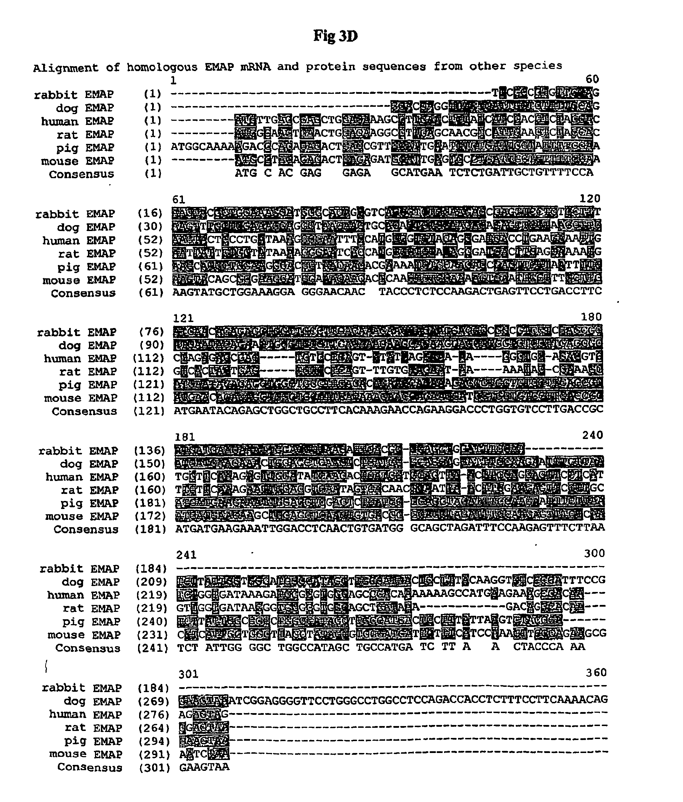

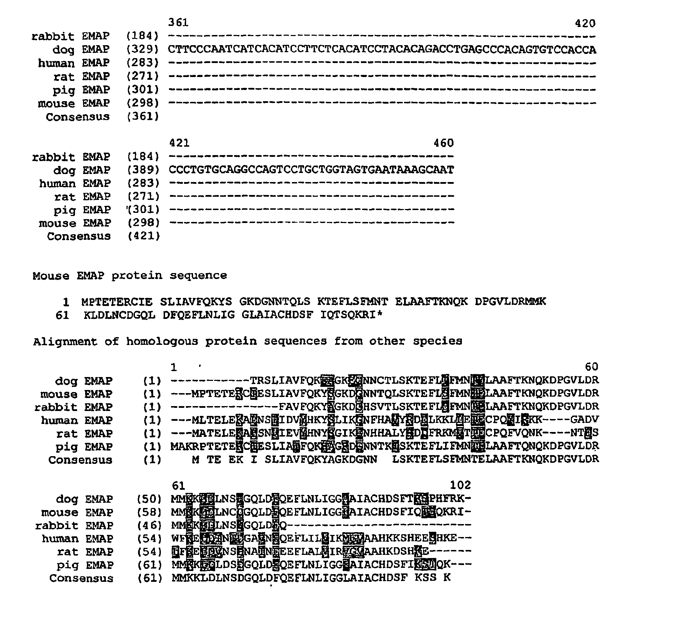

**Figure 3** shows the structure of immunoglobulin enhancer genes. Nucleotide and protein sequence of the alpha-1-antitrypsin and endothelial monocyte-activating polypeptide I gene products.

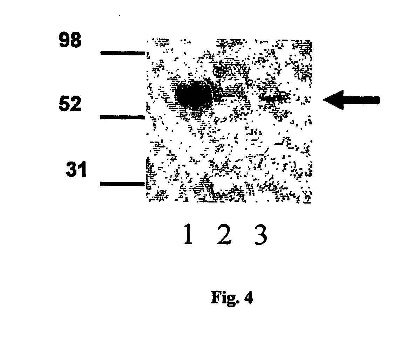

**Figure** 4 shows the use of alpha-1-anti-trypsin antibodies to screen for high-titer antibody producer strains. Supernatant was isolated from H6 parental (lane 1); H34 over-producer strains (lane 2); or H6 high titer producer cells expressing anti-AAT and anti-EMAPI and probed for anti-alpha-1-anti-trypsin. As shown by arrow, a robust extracellular production of alpha-1-anti-trypsin is observed in the low antibody producer line while very little is present in supernatants of high producer strains.

### DETAILED DESCRIPTION OF THE INVENTION

The reference works, patents, patent applications, and scientific literature, including accession numbers to GenBank database sequences that are referred to herein establish the knowledge of those with skill in the art.

Standard reference works setting forth the general principles of recombinant DNA technology known to those of skill in the art include Ausubel et al. CURRENT PROTOCOLS IN MOLECULAR BIOLOGY, John Wiley & Sons, New York (1998); Sambrook et al. MOLECULAR CLONING: A LABORATORY MANUAL, 2D ED., Cold Spring Harbor Laboratory Press, Plainview, New York (1989); Kaufman et al., Eds., HANDBOOK OF MOLECULAR AND CELLULAR METHODS IN BIOLOGY AND MEDICINE, CRC Press, Boca Raton (1995); McPherson, Ed., DIRECTED MUTAGENESIS: A PRACTICAL APPROACH, IRL Press, Oxford (1991).

Methods have been discovered for developing high antibody-producing cells by employing the use of cells or animals with defects in their mismatch repair (MMR) process that in turn results in increased rates of spontaneous mutation by reducing the effectiveness of DNA repair. MMR defective cells or animals are utilized to develop new mutations in a gene of interest. The use of MMR defective cells for production of antibody, immunoglobulin (Ig) gene or derivatives thereof, including cells such as hybridomas; mammalian, plant, yeast or bacterial cells transfected with genes encoding for Ig light and heavy chains or derivatives, can result in subclones that have enhanced production of antibody, immunoglobulin or derivative polypeptides. The process of MMR, also called mismatch proofreading, is carried out by protein complexes in cells ranging from bacteria to mammalian cells (Muller A, Fishel R. (2002) "Mismatch repair and the hereditary non-polyposis colorectal cancer syndrome (HNPCC)" Cancer Invest. 20:102-9). A MMR gene is a gene that encodes for one of the proteins of such a mismatch repair complex. Although not wanting to be bound by any particular theory of mechanism of action, a MMR complex is believed to detect distortions of the DNA helix resulting from non-complementary pairing of nucleotide bases. The non-complementary base on the newer DNA strand is excised, and the excised base is replaced with the appropriate base, which is complementary to the older DNA strand. In this way, cells eliminate many mutations that occur as a result of mistakes in DNA replication.

Dominant negative alleles or inactivation of both alleles by site-specific gene mutation of a given MAR gene can cause a MMR defective phenotype. An example of a dominant negative allele of a MMR gene is the human gene *hPMS2-134,* which carries a truncating mutation at codon 134. The mutation causes the product of this gene to abnormally terminate at the position of the 134th amino acid, resulting in a shortened polypeptide containing the N-terminal 133 amino acids. Such a mutation causes an increase in the rate of mutations, which accumulate in cells after DNA replication. Expression of a dominant negative allele of a mismatch repair gene results in impairment of mismatch repair activity, even in the presence of the wild-type allele. Any allele which produces such effect can be used in this invention. Dominant negative alleles of a MMR gene can be obtained from the cells of humans, animals, yeast, bacteria, or other organisms. Such alleles can be identified by screening cells for defective MAR activity. Moreover, inactivation of both copies of a given MMR gene can also lead to defective MMR. Cells from animals or humans with cancer can be screened for defective mismatch repair. Cells from colon cancer patients may be particularly useful. Genomic DNA, cDNA, or mRNA from any cell encoding a MMR protein can be analyzed for variations from the wild type sequence. Dominant negative alleles or inactivated alleles of a MMR gene can also be created artificially, for example, by producing variants of the *hPMS2-134* allele or other MMR genes. Various techniques of site-directed mutagenesis can be used. The suitability of such alleles, whether natural or artificial, for use in generating hypermutable cells or animals can be evaluated by testing the mismatch repair activity caused by the allele in the presence of one or more wild-type alleles, to determine if it is a dominant negative allele or inactivated allele.

Methods used by those skilled in the art can also be employed to suppress the endogenous activity of a MMR gene resulting in enhanced DNA hypermutability. Such methods employ the use of molecules including but not limited to RNA interference, ribozymes, antisense vectors, somatic cell knockouts, intracellular antibodies, *etc*.

A cell or an animal with defective mismatch repair will become hypermutable. This means that the spontaneous mutation rate of such cells or animals is elevated compared to cells or animals with proficient MMR. The degree of elevation of the spontaneous mutation rate can be at least 2-fold, 5-fold, 10-ford, 20-fold, 50-fold, 100-fold, 200-fold, 500-fold, 1000-fold, or 10,000-fold that of the normal cell or animal. The use of chemical mutagens such as but limited to methane sulfonate, dimethyl sulfonate, 06-methyl benzadine, MNU, ENU, etc. can be used in MMR defective cells to increase the rates an additional 10 to 100 fold that of the MMR deficiency itself.

Transfection is any process whereby a polynucleotide is introduced into a cell. The process of transfection can be carried out in a living animal, *e.g*., using a vector for gene therapy, or it can be carried out *in vitro, e.g.,* using a suspension of one or more isolated cells in culture. The cell can be any type of prokaryotic or eukaryotic cell, including, for example, cells isolated from humans or other primates, mammals or other vertebrates, invertebrates, and single celled organisms such as protozoa, yeast, or bacteria.

In general, transfection will be carried out using a suspension of cells, or a single cell, but other methods can also be applied as long as a sufficient fraction of the treated cells or tissue incorporates the polynucleotide so as to allow transfected cells to be grown and utilized. The protein product of the polynucleotide may be transiently or stably expressed in the cell. Techniques for transfection are well known. Available techniques for introducing polynucleotides include but are not limited to electroporation, transduction, cell fusion, microinjection, the use of calcium chloride, and packaging of the polynucleotide together with lipid for fusion with the cells of interest. Once a cell has been transfected with the candidate gene, the cell can be grown and reproduced in culture. If the transfection is stable, such that the gene is expressed at a consistent level for many cell generations, then a cell line results.

An isolated cell is a cell obtained from a tissue of plants or animals by mechanically separating out individual cells and transferring them to a suitable cell culture medium, either with or without pretreatment of the tissue with enzymes, *e.g*., collagenase or trypsin. Such isolated cells are typically cultured in the absence of other types of cells. Cells selected for the introduction of a candidate Antibody/Ig Enhancer Gene may be derived from a eukaryotic or prokaryotic organism in the form of a primary cell culture or an immortalized cell line, or may be derived from suspensions of single-celled organisms.

Mutant genes in antibody over-producing cells can be detected by analyzing for alterations in the genotype of the cells or animals, for example by examining the sequence of genomic DNA, cDNA, messenger RNA, or amino acids associated with the gene of interest. Mutations can also be detected by screening for the production of antibody or Ig titers. A mutant polypeptide can be detected by identifying alterations in electrophoretic mobility, spectroscopic properties, or other physical or structural characteristics of a protein encoded by a mutant gene. One can also screen for altered function of the protein *in situ,* in isolated form, or in model systems. One can screen for alteration of any property of the cell or animal associated with the function of the gene of interest, such as but not limited to Ig secretion.

Another aspect of the invention is the composition of matter and methods of use whereby two genes, alpha-1-anti-trypsin (AAT) (SEQ ID NO:1) and endothelial monocyte-activating polypeptide I (EMAP) (SEQ ID NO:2) were identified to be significantly suppressed in high titer antibody producer cells. Functional studies have demonstrated that the decreased expression of these genes in parental cell lines using antisense technology can lead to enhanced antibody production. Conversely, the over-expression of these genes in high producer lines that lack robust expression of either the AAT and/or EMAP protein or pathway can suppress antibody expression demonstrating the utility of these genes for regulating antibody production from producer cells.

Yet another aspect of the invention is the regulation of the AAT and/or EMAP protein by biological or chemical agents for the use in modulating their biological pathway for controlling immunoglobulin gene expression in immunological-associated disease states such as allergy and inflammation.

In some embodiments, the invention comprises a host cell which expresses antibody molecules or fragments thereof comprising a defect in the monocyte-activating polypeptide I gene such that expression ofmonocyte-activating polypeptide I is inhibited. These cells may have a defect such as a deletion of monocyte-activating polypeptide I and/or aplha-1-antitrypsin, or a frameshift mutation in one or both of these genes. Alternatively, the host cell may comprise an expression vector comprising an antisense transcript of the monocyte-activating polypeptide I gene and/or alpha-1-antitrypsin gene whereby expression of said antisense transcript suppresses the expression of the gene. In other embodiments, the host cell may comprise a ribozyme that disrupts expression of the monocyte-activating polypeptide I gene or an intracellular neutralizing antibody or antibodies against the monocyte-activating polypeptide I protein and/or alpha-1-antitrypsin protein whereby the antibody or antibodies suppress the activity of the protein(s).

The host cells are useful for expressing antibody molecules in high titer and thus may further comprise polynucleotides encoding fully human antibodies, human antibody homologs, humanized antibody homologs, chimeric antibody homologs, Fab, Fab', F(ab')₂ and F(v) antibody fragments, single chain antibodies, and monomers or dimers of antibody heavy or light chains or mixtures thereof.

The cells of the invention may include mammalian cells, bacterial cells, plant cells, and yeast cells.

For further information on the background of the invention the following references may be consulted:
1. Glaser, V. (1996) Can ReoPro repolish tarnished monoclonal therapeutics? Nat. Biotechol. 14:1216-1217.
2. Weiner, L.M. (1999) Monoclonal antibody therapy of cancer. Semin. Oncol. 26:43-51.
3. Saez-Llorens, X.E. et al. (1998) Safety and pharmacokinetics of an intramuscular humanized monoclonal antibody to respiratory syncytial virus in premature infants and infants with bronchopulmonary dysplasia. Pediat. Infect. Dis. J. 17:787-791.
4. Shield, C.F. et al. (1996) A cost-effective analysis of OKT3 induction therapy in cadaveric kidney transplantation. Am. J. Kidney Dis. 27:855-864.
5. Khazaeli, M.B. et al. (1994) Human immune response to monoclonal antibodies. J. Immunother. 15:42-52.
6. Emery, S.C. and W.J. Harris "Strategies for humanizing antibodies" In: Antibody Engineering C.A.K. Borrebaeck (Ed.) Oxford University Press, N.Y. 1995, pp. 159-183.
7. U.S. Patent No. 5,530,101to Queen and Selick.
8. Reff, M.E. (1993) High-level production of recombinant immunoglobulins in mammalian cells. Curr. Opin. Biotechnol. 4:573-576.
9. Neuberger, M. and M. Gruggermann, (1997) Monoclonal antibodies. Mice perform a human repertoire. Nature 386:25-26.
10. Fiedler, U. and U. Conrad (1995) High-level production and long-term storage of engineered antibodies in transgenic tobacco seeds. Bio/Technology 13:1090-1093.
11. Baker S.M. et al. (1995) Male defective in the DNA mismatch repair gene PMS2 exhibit abnormal chromosome synapsis in meiosis. Cell 82:309-319.
12. Bronner, C.E. et al. (1994) Mutation in the DNA mismatch repair gene homologue hMLH1 is associated with hereditary non-polyposis colon cancer. Nature 368:258-261.
13. de Wind N. et al. (1995) Inactivation of the mouse Msh2 gene results in mismatch repair deficiency, methylation tolerance, hyperrecombination, and predisposition to cancer. Cell 82:321-300.
14. Drummond, J.T. et al. (1995) Isolation of an hMSH2-p160 heterodimer that restores mismatch repair to tumor cells. Science 268:1909-1912.
15. Modrich, P. (1994) Mismatch repair, genetic stability, and cancer. Science 266:1959-1960.
16. Nicolaides, N.C. et al. (1998) A Naturally Occurring hPMS2 Mutation Can Confer a Dominant Negative Mutator Phenotype. Mol. Cell. Biol. 18:1635-1641.
17. Prolla, T.A. et al. (1994) MLH1, PMS 1, and MSH2 Interaction during the initiation of DNA mismatch repair in yeast. Science 264:1091-1093.
18. Strand, M. et al. (1993) Destabilization of tracts of simple repetitive DNA in yeast by mutations affecting DNA mismatch repair. Nature 365:274-276.
19. Su, S.S., R.S. Lahue, K.G. Au, and P. Modrich (1988) Mispair specificity of methyl directed DNA mismatch corrections in vitro. J. Biol. Chem. 263:6829-6835.
20. Parsons, R. et al. (1993) Hypermutability and mismatch repair deficiency in RER+ tumor cells. Cell 75:1227-1236.
21. Papadopoulos, N. et al. (1993) Mutation of a mutL homolog is associated with hereditary colon cancer. Science 263:1625-1629.
22. Perucho, M. (1996) Cancer of the microsatellite mutator phenotype. Biol. Chem. 377:675-684.
23. Nicolaides N.C., K.W. Kinzler, and B. Vogelstein (1995) Analysis of the 5' region of PMS2 reveals heterogenous transcripts and a novel overlapping gene. Genomics 29:329-334.
24. Nicolaides, N.C. et al. (1995) Genomic organization of the human PMS2 gene family. Genomics 30:195-206.
25. Palombo, F. et al. (1994) Mismatch repair and cancer. Nature 36:417.
26. Eshleman J.R. and S.D. Markowitz (1996) Mismatch repair defects in human carcinogenesis. Hum. Mol. Genet. 5:1489-494.
27. Liu, T. et al. (2000) Microsatellite instability as a predictor of a mutation in a DNA mismatch repair gene in familial colorectal cancer. Genes Chromosomes Cancer 27:17-25.
28. Nicolaides, N.C. et al. (1992) The Jun family members, c-JUN and JUND, transactivate the human c-myb promoter via an Ap1 like element. J. Biol. Chem. 267:19665-19672.
29. Shields, R.L. et al. (1995) Anti-IgE monoclonal antibodies that inhibit allergen-specific histamine release. Int. Arch. Allergy Immunol. 107:412-413.
30. Frigerio L. et al. (2000) Assembly, secretion, and vacuolar delivery of a hybrid immunoglobulin in plants. Plant Physiol. 123:1483-1494.
31. Bignami M, (2000) Unmasking a killer: DNA O(6)-methylguanine and the cytotoxicity of methylating agents. Mutat. Res. 462:71-82.
32. Drummond, J.T. et al. (1996) Cisplatin and adriamycin resistance are associated with MutLa and mismatch repair deficiency in an ovarian tumor cell line. J. Biol. Chem. 271:9645-19648.
33. Galio, L. et al. (1999) ATP hydrolysis-dependent formation of a dynamic ternary nucleoprotein complex with MutS and MutL. Nucl. Acids Res. 27:2325-23231.

The above disclosure generally describes the present invention. A more complete understanding can be obtained by reference to the following specific examples which are provided herein for purposes of illustration only.

### EXAMPLE 1: Generation of mismatch repair defective cells for generating enhanced antibody/immunoglobulin producer lines.

Expression of a dominant negative allele in an otherwise MMR proficient cell can render these host cells MMR deficient. The creation of MMR deficient cells can lead to the generation of genetic alterations throughout the entire genome of a host organism's offspring, yielding a population of genetically altered offspring or siblings that may produce biochemicals with altered properties.

It has been discovered that MMR defective cells are useful for creating high-titer antibody-producer cells, including but not limited to rodent hybridomas, human hybridomas, surrogate rodent cells producing human immunoglobulin gene products, surrogate human cells expressing immunoglobulin genes, eukaryotic cells producing single chain antibodies, and prokaryotic cells producing mammalian immunoglobulin genes and/or chimeric immunoglobulin molecules such as those contained within single-chain antibodies. The cell expression systems described above that are used to produce antibodies are well known by those skilled in the art of antibody therapeutics.

To demonstrate the ability to create MMR defective surrogate cell lines and hybridomas using dominant negative alleles of MMR genes, we first transfected a mouse hybridoma cell line (cell line referred to H6) that is known to produce and antibody directed against the IgE protein with an expression vector containing the previously published dominant negative PMS2 mutant referred herein as PMS134 (cell line referred to as H34), or empty vector (cell line referred to as H6vec) or the rodent Chinese hamster ovary (CHO) line (parental referred to as CHO-P and the dominant negative MMR cell referred to as CHO-34). The results showed that the PMS 134 mutant exerts a robust dominant negative effect, resulting in biochemical and genetic manifestations of MMR deficiency as determined by the ability to enhance microsatellite instability of a reporter gene (not shown), which is a hallmark of MMR deficiency as well as increased point mutations that lead to the accumulation of mutations in metabolic genes such as the hypoxanthine phosphoribosyltransferase (HPRT) gene leading to subclones that can grow under selective conditions using methods known by those skilled in the art (Qian Y, Yu Y, Cheng X, Luo J, Xie H, Shen B. Molecular events after antisense inhibition of hMSH2 in a HeLa cell line. Mutat Res 1998 418:61-71). As shown in TABLE 1, CHO cells were preselected to remove spontaneous HPRT mutants that have accumulated over the course of standard propagation and then screened for defected HPRT to determine rate of mutagenesis. Briefly, CHO-P and CHO-34 cells were then grown for 40 doublings and one hundred thousand cells were selected for mutations at the HPRT locus using 6.7ug/ml of 6-thioguanine in growth medium and scored for resistant colonies at day 10. Colony numbers are based out of one million cells screened.

**TABLE 1. HPRT mutations in parental and mismatch repair defective CHO cells**

| **CELL LINE** | **CELLS SCREENED** | **HPRT MUTANTS** |
|---|---|---|
| CHO-P | 1,000,000 | 1+/- 1.7 |
| CHO-34 | 1,000,000 | 62 +/- 10 |

MMR defective cells are now ready to be transfected with immunoglobulin genes and screened to identify subclones with enhanced titer yields or in the case cells already containing expressed immunoglobulin light and heavy chains such as hybridomas, be expanded and screened directly for high titer production lines.

### EXAMPLE 2: Screening of hybridoma clones with increased immunoglobulin production for gene discovery.

An application of the methods presented within this document is the use of MMR deficient hybridomas or MMR defective surrogate cells that can be transfected with immunoglobulin genes such as CHO (see Example 1, Table 1), BHK, NSO, SPO-2, *etc.,* to generate high titer. An illustration of this application is demonstrated within this example whereby the H34 hybridoma, in which a murine MMR-defective cell line producing a mouse IgG monoclonal antibody was grown for 20 generations and clones were isolated in 96-well plates and screened for antibody production. The screening procedure to identify clones that produce high levels of antibody, which is presumed to be due to an alteration within the genome of the host cell line is an assay that employs the use of a plate Enzyme Linked Immunosorbant Assay (ELISA) to screen for clones that produce enhanced antibody titers. 96-well plates containing single cells from H6 parental or H34 pools were grown for 9 days in growth medium (RPMI 1640 plus 10% fetal bovine serum) plus 0.5 mg/ml G418 to ensure clones retain the dominant negative MMR gene expression vector. After 9 days, plates were screened using an anti-Ig ELISA, whereby a 96 well plate is coated with 50uls of conditioned supernatant from independent clones for 4 hours at 4°C. Plates were washed 3 times in calcium and magnesium free phosphate buffered saline solution (PBS^{-/-}) and blocked in 100uls of PBS^{-/-} containing 5% dry milk for 1 hour at room temperature. Plates were then washed 3 times with PBS^{-/-} and incubated for 1 hour at room temperature with 50 uls of a PBS^{-/-} solution containing 1:3000 dilution of a sheep anti-mouse horse radish peroxidase (HRP) conjugated secondary antibody. Plates were then washed 3 times with PBS^{-/-} and incubated with 50 uls of TMB-HRP substrate (BioRad) for 15 minutes at room temperature to detect amount of antibody produced by each clone. Reactions were stopped by adding 50 uls of 500mM sodium bicarbonate and analyzed by OD at 450nm using a BioRad plate reader. Clones exhibiting an enhanced signal over background cells (H6 control cells) were then isolated and expanded into 10 ml cultures for additional characterization and confirmation of ELISA data in triplicate experiments. Clones that produce an increased ELISA signal and have increased antibody levels were then further analyzed for variants that over-express and/or over-secrete antibodies as described in Example 4. Analysis of five 96-well plates each from H6 or H34 cells have found that a significant number of clones with a higher Optimal Density (OD) value is observed in the MMR-defective H34 cells as compared to the H6 controls. Figure 1 shows a representative example of H34 clones producing enhanced levels of antibody. Figure 1 provides primary data from the analysis of 96 wells of fibroblast conditioned medium as negative control (lane 1), H6 (lane 2) or H34 (lane 3) cultures which shows clones from the H34 plate to have a higher OD reading due to genetic alteration of a cell host that leads to over-production/secretion of the antibody molecule.

Clones that produce higher OD values due to enhanced antibody production are sequenced to confirm that mutations have not occurred within the light or heavy chain cDNA. Briefly, 100,000 cells are harvested and extracted for RNA using the Trizol method as described above. RNAs are reverse transcribed using Superscript II as suggested by the manufacturer (Life Technology) and PCR amplified for the full-length light and heavy chains.

These data demonstrate the ability to generate hypermutable hybridomas, or other Ig producing host cells that can be grown and selected, to identify subclones with enhanced antibody/Ig production due to putative structural alterations that have occurred within genome of the host cell that are involved in enhancing antibody production through increased gene expression, protein stability, processing or secretion. Clones can also be further expanded for subsequent rounds of in vivo mutations and can be screened yet higher titer clones due to the accumulation of mutations within additional gene(s) involved in enhancing production. Moreover, the use of chemical mutagens to produce additional genetic mutations in cells or whole organisms can enhance the mutation spectrum in MMR defective cells as compared to "normal" cells. The use of chemical mutagens such as MNU in MMR defective organisms is much more tolerable yielding to a 10 to 100 fold increase in genetic mutation over MAR deficiency alone (Bignami M, (2000) Unmasking a killer: DNA O(6)-methylguanine and the cytotoxicity of methylating agents. Mutat. Res. 462:71-82). This strategy allows for the use of chemical mutagens to be used in MMR-defective antibody producing cells as a method for increasing additional mutations within the host's genome that may yield even higher titer producer strains.

### Example 3: Use of high titer antibody/immunoglobulin producer cells to identify gene involved in enhancing antibody or secreted protein production.

High titer subclones of hybridomas or surrogate antibody/immunoglobulin gene producer cells can be used as a source for gene target discovery to identify genes involved in enhancing antibody titers for use in developing universal high titer production strains for manufacturing and/or for identifying target genes and pathways involved in up or down regulating immunoglobulin production for therapeutic development of immunological disorders such as allergy and inflammation. A benefit of using MMR derived mutants as compared to chemical or ionizing mutagenesis is the observation that cells that are defective for MMR have increased mutation rates yet retain their intact chromosomal profile (Lindor NM, Jalal SM, Van DeWalker TJ, Cunningham JM, Dahl RJ, Thibodeau SN. Search for chromosome instability in lymphocytes with germ-line mutations in DNA mismatch repair genes. Cancer Genet Cytogenet 1998 104:48-51). This feature makes genomic analysis of variants more straightforward because of the decreased background noise that is associated with chemical and radiomutagenesis whereby whole increases and decreases of chromosomal content are associated with the mutagenesis process.

To identify variant gene(s) in high-titer antibody/Ig or derivative producer strains, DNA, RNA and proteins are compared for altered expression or structural patterns used by those skilled in the art. Such techniques employ single polynucleotide analysis (also referred to SNP analysis) which can recognize single nucleotide changes in transcripts of genomic or reverse transcribed RNA templates; microarray or subtractive analysis which can recognize differences in RNA expression profiles; or proteomic analysis which can identify differences in protein profiles between parental and variant lines. Once candidate DNA, transcript or proteins are identified candidates are validated for their role in over-production by: 1.) steady state RNA and/or protein levels and 2.) alteration (over-expression, suppression, and/or introduction of mutant gene) of candidate gene in parental cell line to demonstrate the ability of said candidate gene(s) to recapitulate the over-expression phenotype.

One method for detection of expression patterns among various alternatives, differential expression analysis of H6 parental and H34 high-titer lines, was performed using microarray methods. Analysis of steady state transcripts identified two genes (SEQ ID NO:1 and SEQ ID NO:2) whose expression is suppressed in the high titer H34 cell line. Expression analysis of both genes was carried out using reverse transcriptase coupled polymerase chain reaction (RT-PCR). The putative genes encoded for the murine alpha-1-anti-trypsin (referred to as AAT) (SEQ ID NO:1, accession number 100556; Patent US 4732973; Patent: US 4732973-A2) and the murine endothelial monocyte-activating polypeptide I (referred to as EMAPI) (SEQ ID NO:2 accession number U41341). RNAs were reverse transcribed as described (Nicolaides, N.C. et al. (1995) Genomic organization of the human PMS2 gene family. Genomics 30:195-206). Sense and antisense primers were generated that can specifically amplify the AAT cDNA to yield a 540 bp product and EMAPI cDNA to yield a 272bp product as listed below while the dihydrofolate reductase (DHFR) cDNA was used as a control to monitor RNA integrity and reaction performance using primers as previously described (Nicolaides, N.C., et.al. Interleukin 9: A candidate gene for asthma. 1997 Proc. Natl. Acad. Sci USA 94:13175-13180).

### Primers murine AAT and EMAP expression analysis

SEQ ID NO:3 AAT sense 5'-ttgaagaagccattcgatcc-3'
SEQ ID NO:4 AAT antisense 5'-tgaaaaggaaagggtggtcg-3'
SEQ ID NO:5 EMAPI sense 5'-atgcctacagagactgagag-3'
SEQ ID NO: 6 EMAPI antisense 5'-gattcgcttctgggaagtttgg-3'
PCR reactions were carried out at 95 °C for 30 sec, 58°C for 1 min, 72°C for 1 min for 18 to 33 cycles to measure expression over a linear range. Figure 2 demonstrates a representative profile of steady state expression for the AAT and EMAPI genes in the H6 parental and H34 over-producer strain. As shown, a significant loss of expression was observed in the H34 over producer line for both AAT and EMAPI as compared to the parental control. DHFR expression levels were similar for both samples indicating intact RNA and equal loadings for both samples. These data suggest a roll for AAT and EMAPI in regulating antibody production in mammalian cells.

To confirm that these proteins or lack thereof are involved in regulating antibody production, we have isolated the full-length cDNAs for each gene to be cloned into the sense and/or antisense direction of a mammalian expression vector. Figure 3 shows the isolated cDNA and predicted encoded polypeptide for the murine alpha-1-anti-trypsin (Fig 3A) and the murine endothelial monocyte-activating polypeptide I (Fig 3B). Because of their possible role in regulating antibody or immunoglobulin production in mammalian systems we performed a blast search and identified AAT homologs from hamster (SEQ ID NO:7), human (SEQ ID NO:8), rabbit (SEQ ID NO:9), rat (SEQ ID NO:10), and sheep (SEQ ID NO:11) (Fig 3C) and EMAPI homologs from rabbit (SEQ ID NO:12), dog (SEQ ID NO:13), human (SEQ ID NO:14), rat (SEQ ID NO:15), and pig (SEQ ID NO:16) (Fig 3D) that can be of use for enhancing antibody/immunoglobulin production from cells derived from any of these respective species.

To directly confirm the involvement of AAT and/or EMAPI in regulating antibody production, we generated mammalian expression vectors to produce sense and anti-sense RNAs in parental H6 or over-producer H34 cell lines. If suppression of either or both genes are involved in antibody production, then we would expect enhanced expression in parental lines when treated with antisense vectors that can suppress the AAT and/or EMAP expression levels. Conversely, we should expect to suppress antibody production levels in over producer H34 cells upon reestablished expression of either or both genes. Expression vectors were generated in pUC-based vectors containing the constitutively active elongation factor-1 promoter followed by the SV40 polyA signal. In addition, AAT vectors had a hygromycin selectable marker while EMAP vectors had neomycin selectable markers to allow for double transfection/selection for each vector.

Combinations of antisense AAT and EMAPI vectors were transfected into the parental H6 cell using polyliposomes as suggested by the manufacturer (Gibco/BRL) and stable lines were selected for using 0.5 mg/ml of hygromycinB and the neomycin analog G418. After two weeks of selection, stable clones were derived, expanded and analyzed for sense or antisense gene expression using northern and RT-PCR analysis. Positive clones expressing each vector were then expanded and tested for antibody production using ELISA analysis as described in EXAMPLE 2. Briefly, stable lines or controls were plated at 50,000 cells in 0.2 mls of growth medium per well in triplicates in 96 well microtiter dishes. Cells were incubated at 37°C in 5%CO₂ for 5 days and 50 uls of supernatant was assayed for antibody production. Table 2 shows that H6 cells expressing the antisense AAT and EMAPI produce enhanced levels of antibody in contrast to parental control or H6 cells expressing sense AAT and EMAP1. Conversely, H34 cells (expressing enhanced antibody levels) expressing sense AAT and EMAPI were found to have suppressed antibody production in contrast to H6 parental expressing sense AAT and EMAPI (TABLE 2). These data demonstrate the involvement of AAT and EMAPI in regulating antibody production. Moreover, these data teach us of the use of modulating the expression or function of each of these genes for enhancing or suppressing antibody production for use in developing high titer protein manufacturing strains as well as their use in treating immunological disorders involving hyper or hypo immunoglobulin production.

**TABLE 2. Antisense suppression of AAT and EMAPI results in enhanced antibody production in H6 cells. Restored AAT and EMAPI expression in H34 over-producer cells results in suppressed antibody production.**

| **Cell Line** | **Antibody (ug/ml)** |
|---|---|
| H6 | 13134 +/- 992 |
| H6 AS AAT/EMAP | 29138 +/- 880 |
| H34 | 38452 +/-1045 |
| H34 sense AAT/EMAP | 14421 +/- 726 |

### EXAMPLE 5. Use of small molecules targeted against the alpha-1-anti-tyrpsin pathway for modulating antibody production.

The finding as taught by this application that increasing protease activity via suppressing a natural inhibitor such as alpha-1-antitrypsin may lead to increased antibody production suggests that molecules that alter protease activity may be useful for generating enhanced or suppressed immunoglobulin production from producer lines for use in increasing productivity for manufacturing and/or for use in immunoglobulin regulation of immunological disease. To test the hypothesis, we first used a small molecule protease inhibitor called 4-(2-aminoethyl)-benzenesulfonyl floride (AEBSF), which is a potent trypsin inhibitor (Lawson WB, Valenty VB, Wos JD, Lobo AP. Studies on the inhibition of human thrombin: effects of plasma and plasma constituents. Folia Haematol Int Mag Klin Morphol Blutforsch 1982 109:52-60). Briefly, H34 cells were incubated for 1-3 days in the presence of 4mM AEBSF in 96 well plates and supernatants were tested for antibody production by ELISA. As shown in TABLE 3, H34 cells had a significant suppression of antibody production (0.031 ug/ml) as compared to untreated H34 cells (4.3 ug/ml).

Next, we tested the ability of antiserum directed against AAT (see Example 6 for generation of antiserum) to effect antibody production from H6 lines. If increased protease activity is associated with increased production, then sequestration of a protease inhibitor may increase antibody production. As shown in TABLE 3, H6 parental cells grown in the presence of anti-AAT had increased antibody production (2.6 ug/ml) as compared to H6 cells exposed to preimmune serum (1.6 ug/ml) . These data imply the use of protease activators or inhibitors to modulate antibody production for manufacturing as well as to treat immune disorders associated with hyper or hypo immunoglobulin production.

**TABLE 3. Antibody production from hybridomas incubated with protease inhibitors or inhibitors of natural proteases.**

| **CELL LINE** | **TREATMENT** | **ANTIBODY PRODUCTION UNTREATED** | **ANTIBODY PRODUCTION TREATED** |
|---|---|---|---|
| H34 AEBSF | AEBSF | 4.3 ug/ml | 0.031 ug/ml |
| H6 | PREIMMUNE | - | 1.6 ug/ml |
| H6 | ANTI-ALPHA-1- ANTITRYPSIN | - | 2.6 ug/ml |

### EXAMPLE 6. Use of antibodies to alpha-1-antitrypsin and/or endothelial monocyte-activating polypeptide I for screening of cell clones for enhanced or suppressed immunoglobulin production.

The associated lack of AAT and EMAPI expression with enhanced antibody production from producer strains is useful for screening for high antibody production strains. To demonstrate this utility, we generated monoclonal antiserum against the murine AAT and murine EMAPI protein using polypeptides (SEQ ID NO:17-AAT:(C)QSPIFVGKVVDPTHK and SEQ ID NO: 18-EMAPI (C)IACHDSFIQTSQKRI) derived from their respective translated proteins using methods used by those skilled in the art. We next tested the ability of these antisera to detect protein in the conditioned medium of H6 and H34 cells since both proteins are secreted polypeptides. Briefly, conditioned medium from 10,000 cells were prepared for western blot analysis to assay for steady state protein levels (Figure 4). Briefly, cells were pelleted by centrifugation and 100uls of conditioned supernatant were resuspended in 300 ul of SDS lysis buffer (60 mM Tris, pH 6.8, 2% SDS, 10% glycerol, 0.1 M 2-mercaptoethanol, 0.001% bromophenol blue) and boiled for 5 minutes. Proteins were separated by electrophoresis on 4-12% NuPAGE gels (for analysis of Ig heavy chain. Gels were electroblotted onto Immobilon-P (Millipore) in 48 mM Tris base, 40 mM glycine, 0.0375% SDS, 20% methanol and blocked at room temperature for 1 hour in Tris-buffered saline (TBS) plus 0.05% Tween-20 and 5% condensed milk. Filters were probed with a 1:1000 dilution of mouse anti-AAT or mouse anti-EMAP antiserum in TBS buffer for 1 hour at room temperature. Blots were washed three times in TBS buffer alone and probed with a 1:10000 dilution of sheep anti-mouse horseradish peroxidase conjugated monoclonal antibody in TBS buffer and detected by chemilluminescence using Supersignal substrate (Pierce). Experiments were repeated in duplicates to ensure reproducibility. Figure 4 shows a representative analysis where low producer H6 parental cells (Lane 1) had robust, steady-state AAT protein levels while no expression was observed in H34 over producer cells (Lane 2). These data suggest a method for screening of cell lines for expression of AAT or EMAP to identify high-titer producer strains that can be used to manufacture high levels of antibody or recombinant polypeptides.

The results described above lead to several conclusions. First, the use of mismatch repair defective cells can be used to generate high titer antibody producer cells. Secondly, the generation of high titer producer lines using this method can be used to identify gene(s) involved in increased antibody production. Finally, the methods that can modulate the expression and/or biological activity of the alpha-1-antitrypsin and/or endothelial monocyte-activating polypeptide I can be used to up or down-regulate antibody/immunoglobulin protein production in cells for manufacturing and/or the treatment of immunological-based disorders involving hyper or hype immunoglobulin production (Shields, R.L., et al. (1995) Anti-IgE monoclonal antibodies that inhibit allergen-specific histamine release. Int. Arch Allergy Immunol. 107:412-413).

## Claims

1. An in vitro or ex vivo method for producing a high titer antibody producing cell comprising the step of suppressing the expression of at least one gene involved in antibody production, wherein said gene comprises alpha-1-anti-trypsin.

2. The method of claim 1, whereby said step of suppressing the expression of said gene comprises any one of:
a) introducing an antisense oligonucleotide directed against said alpha-1-antitrypsin gene;
b) introducing an expression vector comprising an antisense transcript directed against said alpha-1-anti-trypsin gene;
c) introducing a knock out targeting vector to disrupt the endogenous function of said alpha-1-antitrypsin gene, or
d) introducing a polynucleotide comprising a ribozyme directed against said alpha-1-antitrypsin gene.

3. The method of claim 1, whereby suppression is achieved by introducing intracellular blocking antibodies against the product of said alpha-1-antitrypsin gene, or by incubating cells with neutralizing antibody or derivates thereof directed against the product of said gene in the growth medium.

4. The method of any preceding claim additionally comprising the step of suppressing the expression of the endothelial monocyte-activating polypeptide I gene.

5. The method of claim 4, whereby said step of suppressing the expression of said endothelial monocyte-activating polypeptide I gene comprises any one of:
a) introducing an antisense oligonucleotide directed against said endothelial monocyte-activating polypeptide 1 gene;
b) introducing an expression vector comprising an antisense transcript directed against said endothelial monocyte-activating polypeptide I gene;
c) introducing a knock out targeting vector to disrupt the endogenous function of said endothelial monocyte-activating polypeptide I, or
d) introducing a polynucleotide comprising a ribozyme directed against said endothelial monocyte-activating polypeptide I gene.

6. The method of claim 4, whereby suppression of said endothelial monocyte-activating polypeptide I gene is achieved by introducing intracellular blocking antibodies against the product of said endothelial monocyte-activating polypeptide I gene, or by incubating cells with neutralizing antibody or derivates thereof directed against the product of said gene in the growth medium.

7. The method of any preceding claim, wherein the cell is a hybridoma, an epithelial cell, ovarian, a kidney cell, a myeloid cell or a lymphoid cell.

8. A host cell which produces antibody molecules or fragments thereof comprising a defect in the alpha-1-antitrypsin gene such that expression of alpha-1-antitrypsin is inhibited.

9. The host cell of claim 8, wherein said defect comprises a deletion of the alpha-1-antitrypsin, or wherein said defect is a frameshift mutation in the alpha-1-antitrypsin gene.

10. The host cell of claim 8, wherein said host cell comprises:
a) an expression vector comprising an antisense transcript of the alpha-1-antitrypsin gene whereby expression of said antisense transcript suppresses the expression of the alpha-1-antitrypsin gene;
b) a ribozyme that disrupts expression of the alpha-1-antitrypsin gene, or
c) an intracellular neutralizing antibody against the alpha-1-antitrypsin protein whereby said antibody suppresses the activity of alpha-1-antitrypsin.

11. The host cell of claim 10, further comprising an expression vector comprising a polynucleotide sequence encoding at least a portion of an antibody molecule.

12. The host cell of claim 11, wherein said polynucleotide encodes at least an immunoglobulin light chain or fragment thereof, or at least an immunoglobulin heavy chain or fragment thereof.

13. The host cell of any of claims 8 to 12 further comprising a defect in the endothelial monocyte-activating polypeptide I gene such that expression of monocyte-activating polypeptide I is inhibited.

14. The host cell of claim 13, wherein said defect comprises a deletion of the endothelial monocyte-activating polypeptide I, or wherein said defect is a frameshift mutation in the endothelial monocyte-activating polypeptide I gene.

15. The host cell of claim 13, wherein said host cell comprises:
a) an expression vector comprising an antisense transcript of the endothelial monocyte-activating polypeptide I gene whereby expression of said antisense transcript suppresses the expression of the endothelial monocyte-activating polypeptide I gene;
b) a ribozyme that disrupts expression of the endothelial monocyte-activating polypeptide I gene; or
c) an intracellular neutralizing antibody against the endothelial monocyte-activating polypeptide I protein whereby said antibody suppresses the activity of endothelial monocyte-activating polypeptide I.

## Patentansprüche

1. Ein in vitro oder ex vivo Verfahren zur Erzeugung einer einen Antikörper von hohem Titer herstellenden Zelle enthaltend den Schritt der Unterdrückung der Expression von wenigstens einem Gen, welches bei der Antikörperherstellung beteiligt ist, wobei besagtes Gen Alpha-1-Antitrypsin beinhaltet.

2. Verfahren nach Anspruch 1, wobei besagter Schritt der Unterdrückung der Expression besagten Gens irgendeinen der folgenden Schritte enthält:
a) Einführung eines gegensinnigen Oligonukleotids, welches gegen gesagtes Alpha-1-Antitrypsin Gen gerichtet ist;
b) Einführung eines Expressionsvectors enthaltend ein gegensinniges Transcript, welches gegen besagtes Alpha-1-Antitrypsin Gen gerichtet ist;
c) Einführung eines Knock-Out Zielvectors um die endogene Funktion besagten Alpha-1-Antitrypsin Gens zu unterbrechen oder
d) Einführung eines Polynukleotids enthaltend ein Ribozyme gerichtet gegen besagtes Alpha-1-Antritrypsin Gen.

3. Verfahren nach Anspruch 1, wobei besagte Unterdrückung erreicht wird durch Einführung intrazellulärer blockierender Antikörper gegen das Produkt besagten Alpha-1-Antitrypsin Gens oder durch Inkubieren der Zellen mit neutralisierendem Antikörper oder Derivaten davon, welche gegen das Produkt besagter Gene im Wachstumsmedium gerichtet ist.

4. Verfahren nach einem der vorangegangenen Ansprüche zusätzlich enthaltend den Schritt der Unterdrückung der Expression der endothelialen Monozyt aktivierenden Polypeptid I Gene.

5. Verfahren nach Anspruch 4, wobei besagter Schritt der Unterdrückung der Expression besagter endothelialer Monozyt aktivierender Polypeptid I Gene wenigstens einen der folgenden Schritte enthält:
a) Einführung eines gegensinnigen Oligonukleotids gerichtet gegen besagte endotheliale Monozyt aktivierende Polypeptid I Gene;
b) Einführung eines Expressionsvectors enthaltend ein gegensinniges Transcript gerichtet gegen besagte endotheliale Monozyt aktivierende Polypeptide I Gene;
c) Einführung eine Knock-Out Zielvectors um die endogene Funktion besagter endothelialer Monozyt aktivierender Polypeptid I Gene zu unterbrechen oder
d) Einführung eines Polynukleotide enthaltend ein Ribozym gerichtet gegen besagte endotheliale Monozyt aktivierende Polypeptid I Gene.

6. Verfahren nach Anspruch 4, wobei besagte Unterdrückung besagter endothelialer Monozyt aktivierender Polypeptid I Gene erreicht wird durch Einführung von intrazellolären blockierenden Antikörpern gegen das Produkt gesagter endothelialer Monozyt aktivierender Polypeptid I Gene oder durch Inkubationszellen mit neutralisierendem Antikörper oder Derivaten davon gerichtet gegen das Produkt besagter Gene in dem Wachstumsmedium.

7. Verfahren nach einem der vorangegangenen Ansprüche, wobei die Zelle eine hybridome Zelle, eine epitheliale Zelle, eine Eierstockzelle, eine Nierenzelle, eine Myeloidzelle oder Lymphoidzelle ist.

8. Gastzelle, die Antikörpermoleküle oder Fragmente davon herstellt enthaltend einen Defekt in dem Alpha-1-Antitrypsin Gen derart, dass die Expression von Alpha-1-Antitrypsin verhindert wird.

9. Gastzelle, nach Anspruch 8, wobei besagter Defekt die Zerstörung des Alpha-1-Antitrypsins enthält oder wobei besagter Defekt eine Frameshift Mutation in dem Alpha-1-Antitrypsin Gen ist.

10. Gastzelle, nach Anspruch 8, wobei besagte Gastzelle enthält:
a) einen Expressionsvector enthaltend ein gegensinniges Transcript des Alpha-1-Antitrypsin Gens, wobei die Expression besagten gegensinnigen Transcripts die Expression des Alpha-1-Antitrypsin Gens unterdrückt;
b) ein Ribozyme, welches die Expression des Alpha-1-Antitrypsin Gens unterbricht oder
c) einen intrazellolären neutralisierenden Antikörper gegen das Alpha-1-Antitrypsin Protein wobei besagter Antikörper die Aktivität von Alpha-1-Antitrypsin unterdrückt.

11. Gastzelle, nach Anspruch 10, weiter enthaltend eine Expressionsvector enthaltend eine Polynukleotid Sequenz, die wenigstens einen Bereich eines Antikörpermoleküls kodiert.

12. Gastzelle, nach Anspruch 11, wobei besagtes Polynukleotid wenigstens eine Immunoglobolin Leichtkette oder ein Fragment von ihr kodiert oder wenigstens eine Immunoglobolin Schwerkette oder ein Fragment von ihr.

13. Gastzelle, nach einem der Ansprüche 8 bis 12, weiterhin enthaltend einen Defekt in dem endothelialen Monozyt aktivierenden Polypeptid I Gen derart, dass die Expression von Monozyt aktivierendem Polypeptid I verhindert wird.

14. Gastzelle, nach Anspruch 13, wobei besagter Defekt eine Zerstörung des endothelialen Monozyt aktivierenden Polypeptid I enthält oder wobei besagter Defekt eine Frameshift Mutation in dem endothelialen Monozyt aktivierenden Polypeptid I Gen ist.

15. Gastzelle, nach Anspruch 13, wobei besagte Gastzelle enthält:
a) einen Expressionsvector enthaltend einen gegensinnigen Transcript des endothelialen Monozyt aktivierenden Polypeptid I Gen, wobei die Expression besagten gegensinnigen Transcripts die Expression des endothelialen Monozyt aktivierenden Polypeptid I Gens unterdrückt;
b) ein Ribozym, welches die Expression des endothelialen Monozyt aktivierenden Polypeptid I Gens unterbricht oder
c) einen intrazellolären neutralisierenden Antikörper gegen das endotheliale Monozyt aktivierenden Polypeptid I Protein, wobei besagter Antikörper die Aktivität von endothelialem Monozyt aktivierenden Polypeptid I unterdrückt.

## Revendications

1. Procédé *in vitro* et *ex vivo* de production d'une cellule produisant des anticorps à un titre élevé comprenant l'étape de suppression de l'expression d'au moins un gène impliqué dans la production d'anticorps, dans lequel ledit gène comprend l'alpha-1-anti-trypsine.

2. Procédé selon la revendication 1, dans lequel ladite étape de suppression de l'expression dudit gène comprend l'une quelconque des opérations suivantes :
a) l'introduction d'un oligonucléotide antisens dirigé contre ledit gène de l'alpha-1-anti-trypsine ;
b) l'introduction d'un vecteur d'expression comprenant un produit de transcription antisens dirigé contre ledit gène de l'alpha-1-anti-trypsine ;
c) l'introduction d'un vecteur ciblé de désactivation pour interrompre la fonction endogène dudit gène de l'alpha-1-anti-trypsine, ou
d) l'introduction d'un polynucléotide comprenant un ribozyme dirigé contre ledit gène de l'alpha-1-anti-trypsine.

3. Procédé selon la revendication 1, dans lequel la suppression est réalisée par l'introduction d'anticorps de blocage intracellulaires dirigés contre le produit dudit gène de l'alpha-1-anti-trypsine, ou par l'incubation des cellules avec un anticorps de neutralisation ou ses dérivés dirigés contre le produit dudit gène dans le milieu de croissance.

4. Procédé selon l'une quelconque des revendications précédentes, comprenant en outre l'étape de suppression de l'expression du gène du polypeptide I d'activation des monocytes endothéliaux.

5. Procédé selon la revendication 4, dans lequel ladite étape de suppression de l'expression dudit gène du polypeptide I d'activation des monocytes endothéliaux comprend l'une quelconque des opérations suivantes :
a) l'introduction d'un oligonucléotide antisens dirigé contre ledit gène du polypeptide I d'activation des monocytes endothéliaux ;
b) l'introduction d'un vecteur d'expression comprenant un produit de transcription antisens dirigé contre ledit gène du polypeptide I d'activation des monocytes endothéliaux ;
c) l'introduction d'un vecteur ciblé de désactivation pour interrompre la fonction endogène dudit polypeptide I d'activation des monocytes endothéliaux, ou
d) l'introduction d'un polynucléotide comprenant un ribozyme dirigé contre ledit gène du polypeptide I d'activation des monocytes endothéliaux.

6. Procédé selon la revendication 4, dans lequel la suppression dudit gène du polypeptide I d'activation des monocytes endothéliaux est réalisée par l'introduction d'anticorps de blocage intracellulaires dirigés contre le produit dudit gène du polypeptide I d'activation des monocytes endothéliaux, ou par l'incubation des cellules avec un anticorps de neutralisation ou ses dérivés dirigés contre le produit dudit gène dans le milieu de croissance.

7. Procédé selon l'une quelconque des revendications précédentes, dans lequel la cellule est un hybridome, une cellule épithéliale, une cellule ovarienne, une cellule de rein, une cellule myéloïde ou une cellule lymphoïde.

8. Cellule hôte qui produit des molécules d'anticorps ou des fragments de celles-ci, comprenant un défaut dans le gène de l'alpha-1-anti-trypsine, de sorte que l'expression de l'alpha-1-anti-trypsine est inhibée.

9. Cellule hôte selon la revendication 8, dans laquelle ledit défaut comprend une délétion dans l'alpha-1-anti-trypsine, ou dans laquelle ledit défaut est une mutation par déphasage dans le gène de l'alpha-1-anti-trypsine.

10. Cellule hôte selon la revendication 8, dans laquelle ladite cellule hôte comprend :
a) un vecteur d'expression comprenant un produit de transcription antisens du gène de l'alpha-1-anti-trypsine, moyennant quoi l'expression dudit produit de transcription antisens supprime l'expression du gène de l'alpha-1-anti-trypsine ;
b) un ribozyme qui interrompt l'expression du gène de l'alpha-1-anti-trypsine, ou
c) un anticorps de neutralisation intracellulaire dirigé contre la protéine alpha-1-anti-trypsine, moyennant quoi ledit anticorps supprime l'activité de l'alpha-1-anti-trypsine.

11. Cellule hôte selon la revendication 10, comprenant en outre un vecteur d'expression comprenant une séquence polynucléotidique codant au moins une partie d'une molécule d'anticorps.

12. Cellule hôte selon la revendication 11, dans laquelle ledit polynucléotide code au moins une chaîne légère d'immunoglobuline ou l'un de ses fragments ou au moins une chaîne lourde d'immunoglobuline ou l'un de ses fragments.

13. Cellule hôte selon l'une quelconque des revendications 8 à 12, comprenant en outre un défaut dans le gène du polypeptide I d'activation des monocytes endothéliaux, de sorte que l'expression du polypeptide I d'activation des monocytes endothéliaux est inhibée.

14. Cellule hôte selon la revendication 13, dans laquelle ledit défaut comprend une délétion dans le polypeptide I d'activation des monocytes endothéliaux, ou dans laquelle ledit défaut est une mutation par déphasage dans le gène du polypeptide I d'activation des monocytes endothéliaux.

15. Cellule hôte selon la revendication 13, dans laquelle ladite cellule hôte comprend :
a) un vecteur d'expression comprenant un produit de transcription antisens du gène du polypeptide I d'activation des monocytes endothéliaux, moyennant quoi l'expression dudit produit de transcription antisens supprime l'expression du gène du polypeptide I d'activation des monocytes endothéliaux ;
b) un ribozyme qui interrompt l'expression du gène du polypeptide I d'activation des monocytes endothéliaux ; ou
c) un anticorps de neutralisation intracellulaire dirigé contre la protéine du polypeptide I d'activation des monocytes endothéliaux, moyennant quoi ledit anticorps supprime l'activité du polypeptide I d'activation des monocytes endothéliaux.
